# EUROPEAN PATENT SPECIFICATION

(11) **EP 3 798 205 B1**
(45) Date of publication and mention of the grant of the patent: **13.04.2022**
(21) Application number: 19199892.1
(22) Date of filing: 26.09.2019
(51) Int. Cl.: C07C 67/313, C09J 133/08, C08F 220/14, C07C 69/73, C08F 222/14, C09J 135/02

(54) **BIO-BASED ACRYLATE MONOMER**
BIOBASIERTES ACRYLATMONOMER
MONOMÈRE D'ACRYLATE À BASE BIO

(43) Date of publication of application: 31.03.2021
(73) Proprietor: Henkel AG & Co. KGaA, 40589 Düsseldorf (DE)
(72) Inventor: Brandt, Adrian, 45219 Essen (DE); Beck, Horst, 41470 Neuss (DE); Nemitz, Ralph, 41363 Jüchen (DE)

(56) References cited:
- WO-A1-2014/172185
- DE-A1-102004 002 279

## Description

### Technical field of the invention

The present invention relates to a bio-based acrylate monomer.

### Background of the invention

(Meth)acrylate adhesives consist primarily of (meth)acrylate monomers and oligomers. There is a constant need for a new (meth)acrylate monomer and oligomer structures having new technical Documents WO 2014/172185 and DE 10 2004 002279 disclose adhesives comprising (meth)acrylates.

For example, low odour and low vapor pressure monomers to improve health and safety of a production line and end users.

These (meth)acrylate monomers and oligomers are traditionally produced using petrol-based raw materials. To increase sustainability, the end users of the (meth)acrylate adhesives are now requesting products with increased bio-based content without compromising performance.

Therefore, there is a need for new (meth)acrylate monomers obtained from increased quantities of bio-based raw material without losing desired physical properties and performance.

### Summary of the invention

The present invention relates to a compound having a structure 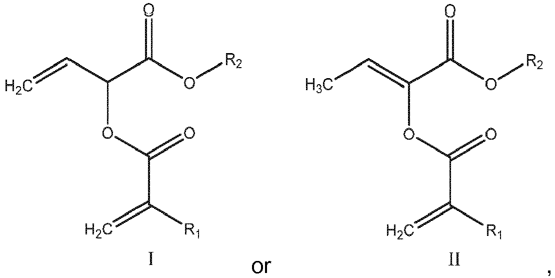 wherein R₁ is H or -CH₃, and wherein R₂ is linear C1-C10 alkyl or branched C3-C10 alkyl. The present invention also relates to a process to produce a compound according to the present invention, wherein bio-based alkyl-2-hydroxy-3-butenoate is reacted with (meth)acryloyl chloride or (meth)acrylic acid or (meth)acrylic anhydride, wherein alkyl-group is linear C1-C10 alkyl or branched C3-C10 alkyl.

The present invention encompasses an adhesive composition comprising a compound according to the present invention.

The present invention further encompasses a use of a compound according to the present invention as a reactive acrylate monomer in a structural bonding adhesive, an acrylic pressure sensitive adhesive, a waterborne acrylic dispersion, an anaerobic acrylic adhesive or a 3D printing resin.

### Detailed description

In the following passages the present invention is described in more detail. Each aspect so described may be combined with any other aspect or aspects unless clearly indicated to the contrary. In particular, any feature indicated as being preferred or advantageous may be combined with any other feature or features indicated as being preferred or advantageous.

In the context of the present invention, the terms used are to be construed in accordance with the following definitions, unless a context dictates otherwise.

As used herein, the singular forms "a", "an" and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps.

The recitation of numerical end points includes all numbers and fractions subsumed within the respective ranges, as well as the recited end points.

All percentages, parts, proportions and then like mentioned herein are based on weight unless otherwise indicated.

When an amount, a concentration or other values or parameters is/are expressed in form of a range, a preferable range, or a preferable upper limit value and a preferable lower limit value, it should be understood as that any ranges obtained by combining any upper limit or preferable value with any lower limit or preferable value are specifically disclosed, without considering whether the obtained ranges are clearly mentioned in the context.

Unless otherwise defined, all terms used in disclosing the invention, including technical and scientific terms, have the meaning as commonly understood by one of the ordinary skill in the art to which this invention belongs to. By means of further guidance, term definitions are included to better appreciate the teaching of the present invention.

The present invention relates to a compound having a structure 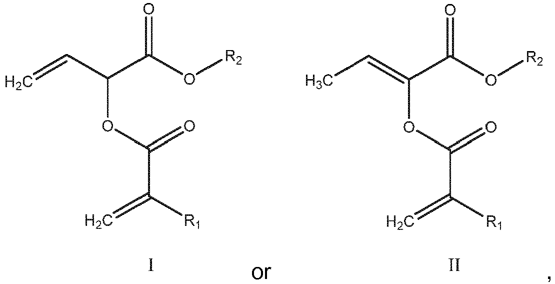 wherein R₁ is H or -CH₃, and wherein R₂ is linear C1-C10 alkyl or branched C3-C10 alkyl.

Preferably, R₁ is -CH₃, and R₂ is selected from methyl-group, ethyl-group and isopropyl-group, preferably R₂ is methyl-group.

In highly preferred embodiment R₁ is -CH₃ and R₂ is -CH₃.

Compounds I and II according to the present invention are bio-based molecules, and therefore desired. In addition, they provide lower shrinkage for adhesive and coating applications. Furthermore, the additional vinyl functionality provides the possibility to perform further modifications, and adjust properties (further cross linking, thiolene reaction and the like). Compounds I and II having R₁ as -CH₃ and R₂ is -CH₃ group are slightly less reactive than compounds I and II having R₁ as H.

Compounds having structure I and II, wherein R₁ is -CH₃ and R₂ is -CH₃ are preferred, because they have a higher boiling point leading to a higher flash point compared to other acrylate monomers like MMA, and therefore, increases the safety aspect of the compound. In addition, compounds having structure I and II, wherein R₁ is -CH₃ and R₂ is -CH₃ have low viscosity, which makes compound easy to handle and easy to use in the adhesive compositions. Furthermore, compound having structure I is particularly preferred because of the more reactive double bond. The vinyl bond is a very important feature, because it can be used for further chemical modification like sulphur or peroxide cross-linking and thiol-ene reaction. In addition, the vinyl bond takes part in the conventional radical mediated cross-linking reaction.

In highly preferred embodiment a compound has a structure 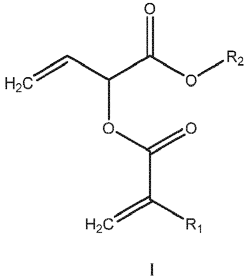 wherein R₁ is -CH₃ and R₂ is -CH₃.

In another highly preferred embodiment, a compound has a structure 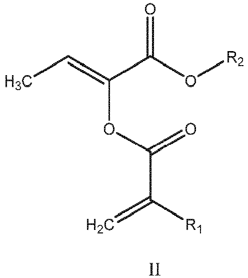 wherein R₁ is -CH₃ and R₂ is -CH₃.

Compound alkyl-2-hydroxy-3-butenoate (1), wherein alkyl (R₂ in structures I and II) is linear C1-C10 alkyl or branched C3-C10 alkyl, preferably selected from methyl-group, ethyl-group and isopropyl-group, and more preferably methyl-group, is used as a starting material to produce compound having structure I or II.

Compound having structure I or II is produced by a process wherein bio-based alkyl-2-hydroxy-3-butenoate (1) is reacted with (meth)acryloyl chloride (2) or (meth)acrylic acid (3) or (meth)acrylic anhydride (4). The reaction is illustrated in scheme 1 below.

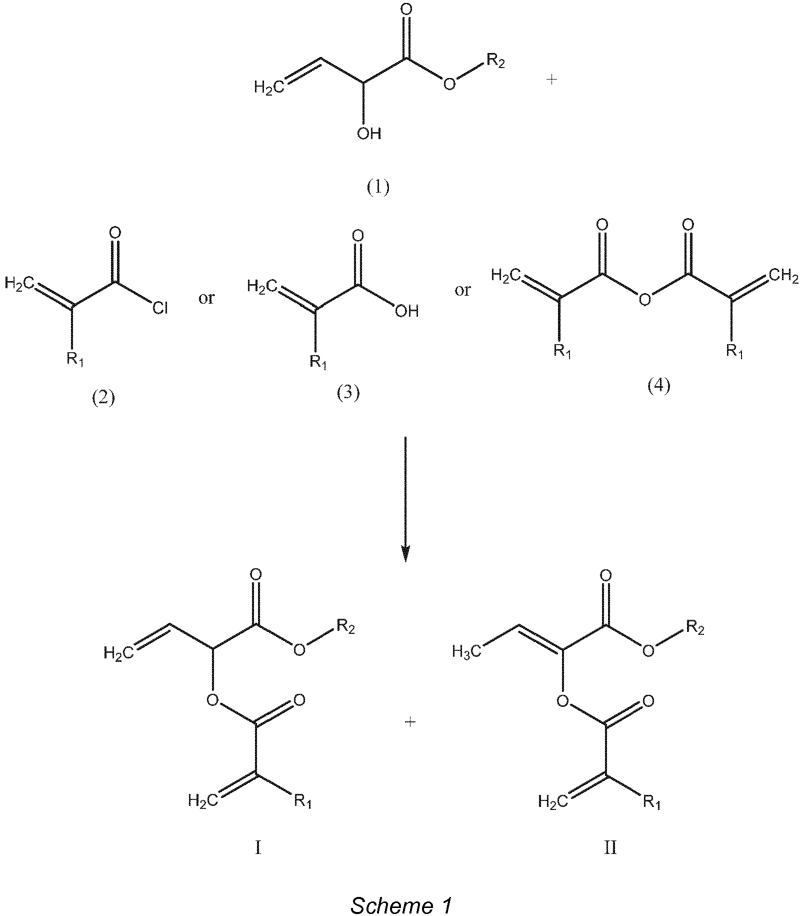

Bio-based alkyl-2-hydroxy-3-butenoate (1) can be obtained from sugar streams. In principle, lactic acid, 2-hydroxy-3-butenoic acid and esters are produced by converting glucose, fructose, surcrose, xylose or glycoaldehyde in a solvent in the presence of solid lewis acid catalyst (details of the process are disclosed in EP2184270). The solvent used in the process drives the formation of the alkyl-group (R₂ in structures I and II). When the reaction is done in methanol alkyl (R₂) is -CH₃, whereas use of ethanol would lead to -CH₂CH₃ group as alkyl (R₂) and isopropyl would lead -CH(CH₃)₂ as alkyl (R₂). If the process is done in bio-based MeOH or biobased EtOH, a 100% bio-based methyl-2-hydroxy-3-butenoate or ethyl-2-hydroxy-3-butenoate can be obtained.

Commercially available methyl-2-hydroxy-3-butenoate is currently available from fine chemical producers in USA, Europe or Asia such as ABCR GmbH or Chemos GmbH.

Suitable commercially available (meth)acryloyl chloride for use in the present invention include but are not limited to methacryloyl chloride from Acros.

Suitable commercially available (meth)acrylic acid for use in the present invention include but are not limited to methacrylic acid from Sigma Aldrich.

Suitable commercially available (meth)acrylic anhydride for use in the present invention include but are not limited to (meth)acrylic anhydride from Sigma Aldrich.

In a preferred embodiment compound having structure I or II is produced by a process wherein bio-based methyl-2-hydroxy-3-butenoate (1) is reacted with (meth)acryloyl chloride (2) or (meth)acrylic acid (3) or (meth)acrylic anhydride (4) in a presence of a catalyst.

Suitable catalyst for use in the present invention is a non-nucleophilic base, preferably from the group consisting of tertiary amine having a structure NR₃, wherein R₃ is an alkyl group, triethyl amine, *N*,*N*-diisopropylethylamine (DIPEA), 1,8-diazabicycloundec-7-ene (DBU), 1,5-diazabicyclo(4.3.0)non-5-ene (DBN), 2,6-Di-tert-butylpyridine, phosphazene bases, such as t-Bu-P₄, Lithium diisopropylamide, sodium bis(trimethylsilyl)amide (NaHMDS), potassium bis(trimethylsilyl)amide (KHMDS), lithium tetramethylpiperidide (LiTMP) and mixtures thereof, preferably triethyl amine, 1,8-diazabicycloundec-7-ene (DBU) and mixtures thereof, more preferably the catalyst is triethyl amine.

The catalyst influences the ratio of structures I and II, which structure is a main product, and which is a side product. Triethyl amine is a preferred catalyst, because it promotes formation of structure I as a main product.

In addition to a selection of a catalyst, reaction conditions and purity of the reagents all influence the ratio of structures I and II.

The reaction is made in an aprotic organic solvent. Suitable solvent for use in the present invention is selected from the group consisting of dichloromethane, hexane, benzene, toluene, 1,4-dioxan, chloroform, diethyl ether, N-methylpyrrolidone, tetrahydrofuran, ethyl acetate, acetone, dimethylformamide, acetonitrile, dimethyl sulfoxide, propylene carbonate and mixtures thereof.

A compound having a structure I or II according to the present invention is a polymerizable monomer and can be used as a polymerizable component for adhesives. Structures I and II can be used either alone or together as a polymerizable component for adhesives.

A compound having a structure I and/or II can be used as a part of an adhesive composition. In one embodiment, an adhesive composition comprises a compound having a structure I and/or II according to the present invention.

One embodiment according to the present invention is directed to an adhesive composition wherein structures I and II are both present, preferably in a ratio I:II from 99.99% : 0.001% to 0.001% : 99.99%.

In one embodiment, an adhesive comprises a compound having a structure I and/or II according to the present invention, an acrylate monomer and/or oligomer, and a radical initiator. The adhesive may be cured by heat and/or UV.

Suitable adhesive may be a structural bonding adhesive such as 2K reactive adhesive; an acrylic pressure sensitive adhesive; an anaerobic acrylic adhesive such as a thread locker; waterborne acrylic dispersions or a 3D printing resin.

A compound having a structure I and/or II can be used as a reactive acrylate monomer in a structural bonding adhesive such as 2K reactive adhesive; an acrylic pressure sensitive adhesive; an anaerobic acrylic adhesive such as a thread locker; waterborne acrylic dispersions or a 3D printing resin.

### Examples

### Example 1

56.2g (0.49 Mol) of methyl-2-hydroxy-3-butenoate (only available from fine chemical producers such as ABCR GmbH or Chemos GmbH) was added into dichloromethane and subsequently treated with 56.3g (0.60 Mol) triethylamine (from Merck), and cooled to 0 - 3 ° C. 60.3g (0.60 Mol) of methacryloyl chloride (from Acros) was added within fifteen minutes, and the mixture was stirred for two hours while the solution turned red. The solvent was evaporated by rotary evaporator, and the concentrate was mixed into dichloromethane and washed with water three times. The organic phase was dried over sodium sulphate and concentrated. The obtained concentrate was dark red, clear viscous substance. The obtained concentrate was mixed with 0.2g of BHT and distilled over short Vigreux column in a high vacuum at max. 95 ° C bath temperature and sample were collected from 50 to 52 °C. Yield 57.2g colourless, water thin liquid having less intensive odour as monomers such as MMA. The structure and purity of the compound was confirmed by NMR. Under these reaction conditions 15% (+/- 3%) of compound II and 85% compound I were obtained.

### Example 2

Compound obtained from Example 1 was mixed with 3 wt.% of Irgacure 2959 (from Ciba Specialty Chemicals. Before mixing, Irgacure2959 was pre-dissolved to acetone. The mixture was heated to remove acetone. Obtained clear solution was placed in aluminium cups and subsequently radiated in the UV chamber for five minutes. The composition was cured to a film with Tg around 64 °C. The cured films were less brittle than films formed from MMA.

Table 1 below lists physical properties of the mixture of compound I (88%) and compound II (12%) according to the present invention. The properties are compared to properties of methyl methacrylate.

Compounds I and II according to the present invention have a higher boiling point resulting in lower vapor pressure leading to better health and safety while handling the compounds; less aggressive smell; lower shrinkage for better adhesion in critical applications; the additional vinyl/double bond functionality provides a further crosslinking/chemical modification possibility; finally due to the different structure one can expect different properties with regard to Tg, adhesion, solubility, compatibility and the like.

**Table 1**

| | Methyl methacrylate (MMA) | Mixture of compound I (88%) and compound II (12%) according to the present invention | Test method |
|---|---|---|---|
| Boiling point (°C) | 101 | 214-226 | A.2 Siedetemperatur of the guideline 67/548/EWG |
| Refractive index | 1.414 | 1.410 | Abbe refractometer |
| Vapour pressure 20°C in mbar | 39.6 | 0.19 | ASTM E 1782-03 |
| Vapour pressure 20°C in mbar | 157 | 1.6 | ASTM E 1782-03 |
| Density of liquid monomer (g/cm³) | 0.944 | 1.064 | DMA 5000 M Density Meter from Anton Paar, "Biegeschwinger"-method |
| Density of solid polymer (g/cm³) | 1.181 | 1.243 | Pyknometer measurements |
| Volume shrinkage in % | 20.39 | 14.4 | Calculations according to different densities. |

## Claims

1. A compound having a structure 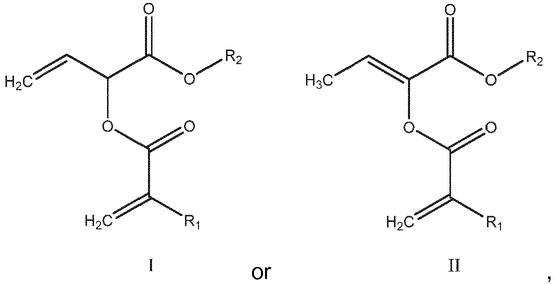 wherein R₁ is H or -CH₃, and wherein R₂ is linear C1-C10 alkyl or branched C3-C10 alkyl.

2. A compound according to claim 1, wherein said R₁ is -CH₃, and wherein R₂ is selected from methyl-group, ethyl-group and isopropyl-group, preferably R₂ is methyl-group.

3. A compound according to claim 1 or 2, wherein said structure is structure I and R₁ is - CH₃ and R2 is -CH₃.

4. A compound according to claim 1 or 2, wherein said structure is structure II and R₁ is - CH₃ and R2 is -CH₃.

5. A process to produce a compound according any of claims 1 to 4, wherein biobased alkyl-2-hydroxy-3-butenoate is reacted with (meth)acryloyl chloride or (meth)acrylic acid or (meth)acrylic anhydride, wherein alkyl is linear C1-C10 alkyl or branched C3-C10 alkyl.

6. A process according to claim 5, wherein said biobased alkyl-2-hydroxy-3-butenoate is reacted with (meth)acryloyl chloride or (meth)acrylic acid or (meth)acrylic anhydride in a presence of a catalyst, wherein alkyl is linear C1-C10 alkyl or branched C3-C10 alkyl.

7. An adhesive composition comprising a compound having a structure I and/or II according to any of claims 1 to 4.

8. An adhesive composition according to claim 7, wherein said structures I and II are both present, preferably in a ratio I:II from 99.99% : 0.001% to 0.001% : 99.99%.

9. An adhesive composition according to claim 7 or 8, wherein said adhesive is structural bonding adhesive, an acrylic pressure sensitive adhesive, an anaerobic acrylic adhesive, acrylic dispersion or a 3D printing resin.

10. A use of a compound according to any of claims 1 to 4 as a reactive acrylate monomer in a structural bonding adhesive, an acrylic pressure sensitive adhesive, an anaerobic acrylic adhesive, waterborne acrylic dispersions or a 3D printing resin.

## Patentansprüche

1. Verbindung, die eine Struktur aufweist 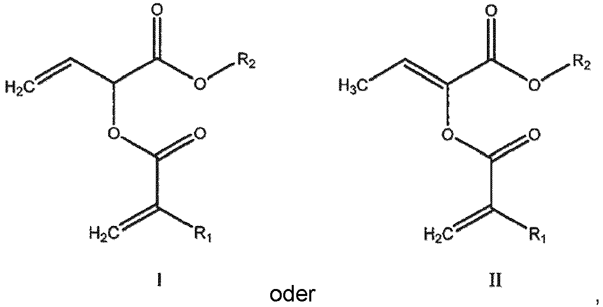 wobei R₁ H oder -CH₃ ist und wobei R₂ lineares C1-C10-Alkyl oder verzweigtes C3-C10-Alkyl ist.

2. Verbindung nach Anspruch 1, wobei das R₁ -CH₃ ist und wobei R₂ aus einer Methylgruppe, einer Ethylgruppe und einer Isopropylgruppe ausgewählt ist, wobei bevorzugt R₂ eine Methylgruppe ist.

3. Verbindung nach Anspruch 1 oder 2, wobei die Struktur Struktur I ist und R₁ -CH₃ ist und R2 -CH₃ ist.

4. Verbindung nach Anspruch 1 oder 2, wobei die Struktur Struktur II ist und R₁ -CH₃ ist und R2 -CH₃ ist.

5. Verfahren, um eine Verbindung nach einem der Ansprüche 1 bis 4 herzustellen, wobei biobasiertes Alkyl-2-hydroxy-3-butenoat mit (Meth)acryloylchlorid oder (Meth)acrylsäure oder (Meth)acrylsäureanhydrid umgesetzt wird, wobei Alkyl lineares C1-C10-Alkyl oder verzweigtes C3-C10-Alkyl ist.

6. Verfahren nach Anspruch 5, wobei das biobasierte Alkyl-2-hydroxy-3-butenoat mit (Meth)acryloylchlorid oder (Meth)acrylsäure oder (Meth)acrylsäureanhydrid in einer Gegenwart eines Katalysators umgesetzt wird, wobei Alkyl lineares C1-C10-Alkyl oder verzweigtes C3-C10-Alkyl ist.

7. Klebstoffzusammensetzung, die eine Verbindung umfasst, die eine Struktur I und/oder II gemäß einem der Ansprüche 1 bis 4 aufweist.

8. Klebstoffzusammensetzung nach Anspruch 7, wobei die Strukturen I und II beide gegenwärtig sind, bevorzugt in einem Verhältnis I : II von 99,99 % : 0,001 % bis 0,001 % : 99,99 %.

9. Klebstoffzusammensetzung nach Anspruch 7 oder 8, wobei der Klebstoff ein struktureller verklebender Klebstoff, ein druckempfindlicher Acrylklebstoff, ein anaerober Acrylklebstoff, eine Acryldispersion oder ein 3D-Druckharz ist.

10. Verwendung einer Verbindung nach einem der Ansprüche 1 bis 4 als ein reaktives Acrylatmonomer in einem strukturellen verklebenden Klebstoff, einem druckempfindlichen Acrylklebstoff, einem anaeroben Acrylklebstoff, einer wässrigen Acryldispersionen oder einem 3D-Druckharz.

## Revendications

1. Composé ayant une structure 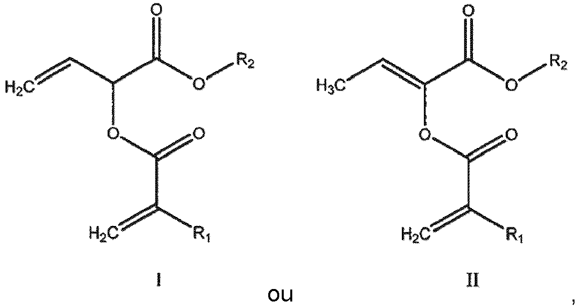 R₁ étant H ou -CH₃, et R₂ étant un alkyle linéaire en C1 à C10 ou un alkyle ramifié en C3 à C10.

2. Composé selon la revendication 1, ledit R₁ étant -CH₃ et R₂ étant choisi parmi un groupe méthyle, un groupe éthyle et un groupe isopropyle, de préférence R₂ étant un groupe méthyle.

3. Composé selon la revendication 1 ou 2, ladite structure étant la structure I et R₁ étant - CH₃ et R₂ étant -CH₃.

4. Composé selon la revendication 1 ou 2, ladite structure étant la structure II et R₁ étant - CH₃ et R₂ étant -CH₃.

5. Procédé destiné à produire un composé selon l'une quelconque des revendications 1 à 4, l'alkyl-2-hydroxy-3-buténoate biosourcé étant mis à réagir avec du chlorure de (méth)acryloyle ou de l'acide (méth)acrylique ou de l'anhydride (méth)acrylique, l'alkyle étant un alkyle linéaire en C1 à C10 ou un alkyle ramifié en C3 à C10.

6. Procédé selon la revendication 5, ledit alkyl-2-hydroxy-3-buténoate biosourcé étant mis à réagir avec du chlorure de (méth)acryloyle ou de l'acide (méth)acrylique ou de l'anhydride (méth)acrylique en présence d'un catalyseur, l'alkyle étant un alkyle linéaire en C1 à C10 ou un alkyle ramifié en C3 à C10.

7. Composition adhésive comprenant un composé ayant une structure I et/ou II selon l'une quelconque des revendications 1 à 4.

8. Composition adhésive selon la revendication 7, lesdites structures I et II étant toutes deux présentes, de préférence dans un rapport I:II de 99,99 % : 0,001 % à 0,001 % : 99,99 %.

9. Composition adhésive selon la revendication 7 ou 8, ledit adhésif étant un adhésif de liaison structurelle, un adhésif acrylique sensible à la pression, un adhésif acrylique anaérobie, une dispersion acrylique ou une résine d'impression 3D.

10. Utilisation d'un composé selon l'une quelconque des revendications 1 à 4 en tant que monomère acrylate réactif dans un adhésif de liaison structurelle, un adhésif acrylique sensible à la pression, un adhésif acrylique anaérobie, des dispersions acryliques en phase aqueuse ou une résine d'impression 3D.
